# EUROPEAN PATENT SPECIFICATION

(11) **EP 0 578 620 B1**
(45) Date of publication and mention of the grant of the patent: **18.04.2001**
(21) Application number: 93830270.0
(22) Date of filing: 24.06.1993
(51) Int. Cl.: A61K 31/00, A61K 9/06, A61K 31/40

(54) **Pharmaceutical compositions for transdermal delivery of melatonin and/or its analogues**
Pharmazeutische Zusammensetzungen zur transdermalen Verabreichung von Melatonin und/oder seine Analoge
Composés pharmaceutiques pour la délivrance transdermale de la mélatonine et/ou ses analogues

(30) Priority: 24.06.1992 IT MI921556
(43) Date of publication of application: 12.01.1994
(73) Proprietor: I.F.L.O. S.a.s. di Giorgio e Aldo Laguzzi, I-20127 Milano (IT)
(72) Inventor: Borgonovo, Margherita, I.F.L.O. S.a.s. di Giorgio, I-20127 Milano (IT); Introini, Carlo, I.F.L.O. S.a.s. di Giorgio, I-20127 Milano (IT); Fraschini, Franco, I.F.L.O. S.a.s. di Giorgio, I-20127 Milano (IT); Stankov, Bojidar, I-20129 Milano (IT)
(74) Representative: Cicogna, Franco

(56) References cited:
- EP-A- 0 518 468
- WO-A-86/05093
- WO-A-87/00432
- WO-A-93/07870
- DATABASE WPI Derwent Publications Ltd., London, GB; AN 86-289026 & JP-A-61 212 512 (SHISEIDO KK) 20 September 1989
- DATABASE WPI Derwent Publications Ltd., London, GB; AN 93033597 & JP-A-61 221 106 (SHISEIDO KK) 10 January 1986

## Description

The present invention relates to melatonin agonists, more specifically to pharmaceutical compositions which are very efficient in the treatment of pathologies which interfere with the circadian rhythms.

Under conditions of the natural environment, the endogenous biological rhythms are adapted to the regular alternation of day and night and to the slower cycles, months and seasons.

Human seasonality is clearly perceptible and under certain circumstances extremely important, for instance, seasonal depression, in spite of the universal use of heating and artificial light in developed countries.

Man has remained as a daily species but social organization frequently involves a lack of correspondence between our physiology and our environment.

Melatonin (N-acetyl-5-Methoxy tryptamine) is a hormone rhythmically secreted by the pineal gland of vertebrates with high concentration during the night hours both in the same gland as well as in the cephaloarakidian liquid and in the peripheral blood.

The synthesis and the secretion of melatonin by the pineal gland is characterized by a circadian rhythm controlled by an endogenous biological clock.

The phase of the rhythm is synchronized to the photo-period through the light and consequently melatonin is considered one of the main transducers of the seasonal photo-periodic information. In fact, the most important function of this hormone is of influencing the reproductive ability in animals which are highly photo-periodic.

Recent studies, however, have shown other sites and other mechanisms of action of melatonin connected to a synchronization necessary for the expression of circadian endogenous rhythms. Therefore, it is possible to assume that melatonin is involved in pathologies which interfere with circadian rhythms (chrono-pathologies).

In the last few years, the discovery and the description of receptors having high affinity for melatonin in the suprachiasmatic nucleus, (SCN) of vertebrates have given substantial stimulation to research in this field. There are described sites and mechanisms of action at the cellular level in this indole derivative, analogs have been synthesized and experimental models of the evaluation of their activity have been made.

At present new agonists of melatonin, specifically 2-iodo-5-methoxy and 2-bromo-5-methoxy are available. The possible applications of melatonin or its potent antagonists in actual clinical practice are:

### 1) Disorders and pathologies of the circadian rhythm (chrono pathologies)

The main applications in this field are:

### 1. a. Jet-lag

The most substantial desynchronization is observed in those individuals who go through more than 5 time zones, particularly in an eastern direction.

Treatment with melatonin brings about an improvement of the syndrome, in spite of the fact that there are found some problems connected with different absorption from individual to individual (employed doses).

### 1. b. Disturbances of sleep such as "Delayed Phase Sleep Syndrome" and disorders of the temporal organization, both macro and micro structural organization of sleep.

Melatonin should be, therefore, an important adjuvant in the therapy with benzodiazepine, Benzodiazepines derivatives administered during the off-phase determine the delay of the same phase with a consequent interruption of the circadian rhythms. This action is due, in addition to the action at the level of the neuro-thalamocortical network, to a direct influence on the internal biological clock (suprachiasmatic nucleus, SCN).

Very frequently also the endogenous rhythm of melatonin is altered. The benzodiazepine derivatives exhibit also substantial collateral action such as paradoxical repercussions such as anxiety and suicidal impulses.

Receptors of melatonin having high density and affinity are present in the SCN of mammals including man. Melatonin administered simultaneously to benzodiazepine (BDZ) causes a shifting of the phase, thus compensating the activity of the benzodiazepine derivatives. Melatonin by itself is in condition of improving the temporal organization of sleep influencing the macro and micro-structure interacting with the complex of the receptor Gaba.

### 1. c. Working people subject to different schedules.

People who carry out work with a rotation of their schedule frequently suffer from disturbances of the circadian rhythm which manifest themselves with sleep/awake cycles, insomnia, hypoactivity during the day and depression. Frequently, also, the endogenous rhythm of melatonin is out of phase.

### 1. d. Treatment of people who don't see and who are desynchronized

Patients who are not capable of seeing light suffer frequently from disturbances connected with their state of "free running" circadian rhythmicity. In more than 85% of cases which have been studied, the desynchronization is obtained by administering the melatonin during the off-phase causing an advance of the phase.

### 1. e. Depressive Seasonal Syndrome (SAD)

The delay of the phase observed in the majority of patients affected by SAD has been corrected by exposing patients in the morning hours to intense light which causes advance of the phase. In these patients, it has been observed that the rhythm of melatonin is abnormal. For this reason melatonin can be used as additional therapy (treatment during the night hours in low doses) in order to increase the shifting of the phase. Experiments in this type have not been carried out.

### 2. Advance of Age

With advance of age, concentrations of melatonin both in the serum as well as in the pineal gland decrease as a result of the degeneration of the same gland. The several problems connected with the dysfunction of the gland in a mature individual can probably be considered due to an alteration of the circadian rhythm, particularly connected also to an altered sensitivity to light.

### The main applications are:

### 2. a. Alterations of circadian rhythm

### 2. b. Sleep disturbances

### 3. Immunological deficit

In the last few years the immunomodulating abilities of melatonin have been demonstrated utilizing several in vivo and in vitro models.

Melatonin and its agonists are capable of increasing the chemiotaxis of human monocytes isolated from peripheral blood, thus showing a direct involvement of the transduction of the signal between cell and cell in the immunological system.

The action of melatonin is in relation to the period of administration; therefore, again it is evident that the indole derivative exhibits an influence on the circadian organization, this time in terms of immunocompetency.

### 4. Neoplasias

Together with a few neoplastic pathologies, there are observed altered concentration of melatonin in the peripheral blood. The direct action of melatonin and its agonists in decreasing the cellular proliferation has been shown in vitro in a few neoplastic cellular locations (human melanoma and murino, cells of mammary carcinoma). The studies in vivo, carried out in several species, have shown that melatonin and/or its agonists may be administered in the treatment supplemental to chemotherapy. The action of melatonin in vivo can be due to its positive influences on the immunological system.

The most serious problem connected with the utilization of melatonin and its agonists is the route of administration. Melatonin has a very short half life (about 30 minutes in man). The bio-availability of endogenous melatonin is about 6-8 hours with high hematic levels (100-200 pg/cc) during the night of the subjects.

This phenomenon is due to the synthesis and continuous release of melatonin by the pineal gland during the period of darkness.

In order to obtain the plasmatic levels comparable for a period of time in the case of oral or parenteral application of exogenous melatonin, very high doses of melatonin must be administered (80-100 mg/dose). A similar treatment determines the achievement of excessive concentrations during the first hours (60-150 minutes after administration) with a quick decrease afterwards.

Another problem is connected with the differences from individual to individual in the absorption.

The hematic levels in man after the administration of melatonin in doses in the range of 2.5-100 mg may have differences from individual to individual even more than 100 times.

Through profound studies carried out in the last few years, it has been clearly shown that the period of bio-availability of melatonin (or its agonists) is extremely important in order to obtain substantial biological responses.

The central nervous system responds in direct manner to the changes in the length of the signal of melatonin rather than the amplitude.

Studies carried out with intravenous administration of melatonin under conditions of continuous infusion have shown the importance of the continuous bioavailability vs. the short signal of great amplitude. In any event, from a therapeutic point of view, it is inconceivable to utilize a substance under conditions of continuous controlled infusion. These are the main reasons for the fact that substantial utilization of melatonin or its powerful agonists in human clinical studies are lacking and for this reason other alternative routes of administration have been sought.

The object of the present invention is to solve the problem discussed hereinabove carrying out the administration of melatonin agonists in such a manner as to obtain as much as possible the typical characteristics of the natural production of melatonin by the human body.

Within the scope of the present invention, a particular object is to carry out an administration of melatonin agonists which permits to achieve controllable hematic levels in an interval corresponding to the endogenous levels of melatonin.

Another object of the present invention is to carry out the administration of melatonin agonists in a manner which permits to achieve bioavailable hematic levels for prolonged controlled periods of time.

The objects mentioned hereinabove as well as others which will be clearly shown hereinbelow, are achieved by administration of melatonin and its agonists particularly in the pathologies which interfere with the circadian rhythms, the administration being carried out percutaneously by application of compositions which contain melatonin and/or its agonists in a percentage between 0.1 and 5% v/v.

Experimental tests have shown that the percutaneous administration of melatonin and/or its agonists exhibits a certain potency and may be carried out by means of oils, gels, pastes, or creams by means of solid supports such as patch, occluded or non-occluded gauzes.

The quantity of melatonin and/or its agonists (2I-melatonin, 2Br-melatonin, 2Cl-melatonin, 2F-melatonin, 2Br-6Cl melatonin, 2I,6-Cl-melatonin, etc.) which must be inserted in the basic formulation, may vary between 0.1 up to 5% v/v as a function of the hematic doses which one wishes to achieve and as a function of the period of administration and the variations in the structure of the agonists which may be used are reported in the formulations hereinbelow (wherein the percentages of the various ingredients are to be considered as expressed in weight): 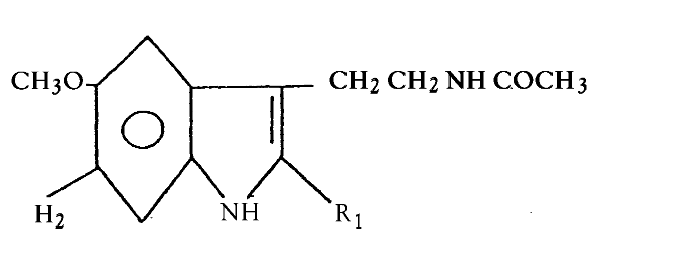 in which R₁ = Cl, I, Br, F.

The formulations and the concentration of melatonin agonists may vary also as a function of the excipients and penetration modulators. Some examples are reported hereinbelow:

| Gel 1 | |
|---|---|
| Homopolymer of acrylic acid | 4 |
| Allyl ether of sugars | 4 |
| Ethanol | 50 |
| Melatonin | 1 |
| Imidazolidinylurea | 3 |
| Glycerin | 20 |
| Triethanolamine | 30 |
| Hydrogenated castor oil | |
| Polyethylene glycol (OE = 40) | 2 |
| Cyclodimethylpolysiloxane | 1 |
| Benzoate of fatty alcohols (aliphatic) | 1 |
| Isodamascone | 0,3 |
| Deionized water | 1000 |

| Gel 2 | |
|---|---|
| Homopolymer of acrylic acid | 4 |
| Allyl ether of sugars | 4 |
| Ethyl alcohol | 320 |
| Melatonin | 10 |
| Imidazolidinylurea | 3 |
| Glycerin | 20 |
| Triethanolamine | 10 |
| Hydrogenated castor oil | |
| Polyethylene glycol (OE = 40) | 2 |
| Cyclodimethylpolysiloxane | 1 |
| Benzoate of fatty alcohols (aliphatic) | 1 |
| Isodamascone | 0,3 |
| Deionized water | 1000 |

| Gel 3 | |
|---|---|
| Homopolymer of acrylic acid | 4 |
| Allyl ether of sugars | 4 |
| Ethanol | 303 |
| Melatonin | 30.3 |
| Phospholipids | 91 |
| Imidazolidinylurea | 3 |
| Glycerin | 20 |
| Triethanolamine | 10 |
| Hydrogenated castor oil | |
| Polyethylene glycol (OE = 40) | 2 |
| Cyclodimethylpolysiloxane | 1 |
| Benzoate of fatty alcohols (aliphatic) | 1 |
| Isodamascone | 0.3 |
| Deionized water | 1000 |

| Gel 4 | |
|---|---|
| Polyglyceryl methacrylate | 350 |
| Ethanol | 50 |
| Melatonin | 1 |
| Hydrogenated castor oil | |
| Polyethylene glycol (DE = 40) | 5 |
| 2-phenoxyethanol | 2 |
| Imidazolidinylurea | 3 |
| Deionized water | 1000 |

| Oil 1 | |
|---|---|
| Cyclodimethylpolysiloxane | 478 |
| Dimethylpolysiloxane | 250 |
| Isostearyl palmitate | 250 |
| Tocopheryl acetate | 20 |
| Melatonin | 1 |
| 2-phenoxyethanol | 2 |

| Emulsion Oil/Water | |
|---|---|
| Alkylesters of: | |
| Polyethylene glycol (OE = 8) | 100 |
| Cetyl and stearyl alcohol | 15 |
| Glyceryl tristearate | 30 |
| Hydrogenated glycerides of tallow | 5 |
| Alkyl benzoate | 70 |
| Jojoba oil | 15 |
| Melatonin | 1 |
| Dimethylpolysiloxane | 10 |
| Tocopheryl acetate | 10 |
| Magnesium ascorbyl phosphate | 5 |
| Glycerin | 30 |
| 2-phenoxyethanol | 2 |
| Imidazolidinylurea | 3 |
| Deionized water | 1000 |

| Emulsion A/S O 1 | |
|---|---|
| Lauryl dimethylpolysiloxane | |
| Polyethylene glycol copoliolo | 20 |
| Dimethylpolysiloxane | 100 |
| Cyclicdimethylpolysiloxane | 50 |
| Melatonin | 1 |
| Tocopheryl acetate | 2 |
| Alkyl lactate | 47 |
| Polyethylene glycol lactate (OE = 3) | 5 |
| Sodium chloride | 20 |
| Glycerin | 20 |
| Imidazolidinylurea | 2 |
| Deionized water | 1000 |

| Emulsion A/S-O 2 | |
|---|---|
| Cetyldimethylpolysiloxane | |
| Polyethylene glycol copoliolo | |
| cetyldimethylpolysiloxane | |
| polyglyceryl oleate and hexyl laurate | 50 |
| Polyisoprene | 140 |
| Dimethylpolysiloxane | 20 |
| Cyclicdimethylpolysiloxane | 30 |
| Tocopheryl acetate | 1 |
| Melatonin | 1 |
| Sodium chloride | 20 |
| 2-phenoxyethanol | 2 |
| Imidazolidinylurea | 3 |
| Sorbitol | 20 |
| Deionized water | 1000 |

In all the above mentioned excipients, melatonin derivatives may be inserted together with a carrier and combined with:
1) Phospholipids of liposomic structure, niosomic and non-niosomic structure
2) Solubilizing agents
3) Kappa elastin (elastic fraction of the protein)
4) Proteins or peptides

Melatonin derivatives may be combined with penetration retardants such as the proteic microspherules of cellulose or other material for the purpose of better modulating the transcutaneous introduction on the basis of the requirements and the therapeutic necessities. The pharmaceutical compositions used in the present studies have clearly demonstrated that the transcutaneous administration of melatonin agonists may be extremely useful.

The hematic levels of the substances reached after transcutaneous administration with controlled concentration correspond very well to the hematic physiological levels of endogenous melatonin in men.

By varying applied concentrations, the levels in the peripheral blood may be controlled in the desired range as it is clearly shown in the following table.

**TABLE 1**

| Doses of melatonin applied by the transcutaneous route (mg) and average concentrations in the peripheral blood reached (pg/ml) in healthy volunteers individuals during the photo phase (about 12 hours) | | | | | | | |
|---|---|---|---|---|---|---|---|
| Time | 0 | 15' | 30' | 60' | 120' | 240' | 300' |
| Dose | Concentration (pg/ml) | | | | | | |
| 5 mg | 45 | 811 | 1148 | 1276 | 1119 | 1110 | 987 |
| 3 mg | 38 | 398 | 732 | 812 | 732 | 702 | 611 |
| 0.5 mg | 44 | 70 | 177 | 179 | 169 | 180 | 158 |

By application through the transcutaneous route of different concentrations of melatonin or its agonists (0.5-5 mg) the hematic levels obtained correspond very well to a correlation between the applied doses/achieved concentration which is very high (r=0.89, p 0.01).

Of great importance must be considered the fact that following the transcutaneous administration, the levels of melatonin or its agonists in the peripheral blood are very stable for a prolonged period of time (4-6 hours) thus simulating the natural condition.

This manner of administration permits to achieve the two essential conditions for the purpose of achieving valuable biological responses:
1) controllable hematic levels in a range corresponding to the endogenous levels of melatonin;
2) hematic levels which are bioavailable for prolonged controlled periods of time,

## Claims

1. A pharmaceutical composition for transcutaneous administration particularly effective in the treatment of pathologies which interfere with the circadian rhythms, which comprises as the active ingredient one or more melatonin agonists according to the formula hereinbelow: 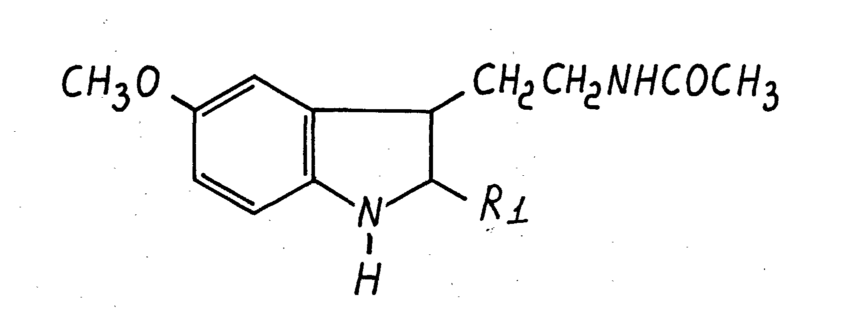 selected from:
2-Br-N-acetyl-5-methoxytryptamine
2-I-N-acetyl-5-methoxytryptamine
2-F-N-acetyl-5-methoxytryptamine
2-C1-N-acetyl-5-methoxytryptamine, wherein the percentage of said active ingredient is between 0.1 and 5% v/v.

2. The pharmaceutical composition according to claim 1 wherein said composition is in the form of an oil.

3. The pharmaceutical composition according to claim 1 wherein said composition is in the form of a gel.

4. The pharmaceutical composition according to claim 1 wherein said composition is in the form of a paste or cream.

5. The pharmaceutical composition according to claim 1 wherein said composition is applied on a solid support.

6. The pharmaceutical composition according to claim 5 wherein said solid support is patch, occluded or non-occluded gauze.

7. The pharmaceutical composition according to claim 1 wherein said composition additionally comprises a penetration retardant.

8. The pharmaceutical composition according to claim 7 wherein said penetration retardants are proteic microspherules of cellulose .

9. The pharmaceutical composition for transcutaneous administration in the form of a gel which consists of:
| | |
|---|---|
| Homopolymer of acrylic acid | 4 |
| Allyl ether of sugars | 4 |
| Ethanol | 50 |
| Melatonin | 1 |
| Imidazolidinylurea | 3 |
| Glycerin | 20 |
| Triethanolamine | 30 |
| Hydrogenated castor oil | |
| Polyethylene glycol (OE = 40) | 2 |
| Cyclodimethylpolysiloxane | 1 |
| Benzoate of fatty alcohols (aliphatic) | 1 |
| Isodamascone | 0,3 |
| Deionized water | 1000 |
wherein the percentages of the various ingredients are to be considered as expressed in weight.

10. The pharmaceutical composition for transcutaneous administration particularly effective in the treatment of pathologies which interfere with the circadian rhythms which consists of:
| | |
|---|---|
| Homopolymer of acrylic acid | 4 |
| Allyl ether of sugars | 4 |
| Ethyl alcohol | 320 |
| Melatonin | 10 |
| Imidazolidinylurea | 3 |
| Glycerin | 20 |
| Triethanolamine | 10 |
| Hydrogenated castor oil | |
| Polyethylene glycol (OE = 40) | 2 |
| Cyclodimethylpolysiloxane | 1 |
| Benzoate of fatty alcohols (aliphatic) | 1 |
| Isodamascone | 0,3 |
| Deionized water | 1000 |
wherein the percentages of the various ingredients are to be considered as expressed in weight.

11. A pharmaceutical composition for transcutaneous administration which consists of:
| | |
|---|---|
| Homopolymer of acrylic acid | 4 |
| Allyl ether of sugars | 4 |
| Ethanol | 303 |
| Melatonin | 30.3 |
| Phospholipids | 91 |
| Imidazolidinylurea | 3 |
| Glycerin | 20 |
| Triethanolamine | 10 |
| Hydrogenated castor oil | |
| Polyethylene glycol (OE = 40) | 2 |
| Cyclodimethylpolysiloxane | 1 |
| Benzoate of fatty alcohols (aliphatic) | 1 |
| Isodamascone | 0.3 |
| Deionized water | 1000 |
wherein the percentages of the various ingredients are to be considered as expressed in weight.

12. A pharmaceutical composition for transcutaneous administration which is in the form of a gel consisting of:
| | |
|---|---|
| Polyglyceryl methacrylate | 350 |
| Ethanol | 50 |
| Melatonin | 1 |
| Hydrogenated castor oil | |
| Polyethylene glycol (DE = 40) | 5 |
| 2-phenoxyethanol | 2 |
| Imidazolidinylurea | 3 |
| Deionized water | 1000 |
wherein the percentages of the various ingredients are to be considered as expressed in weight.

13. A pharmaceutical composition for transcutaneous administration which is in the form of an oil.
| | |
|---|---|
| Cyclodimethylpolysiloxane | 478 |
| Dimethylpolysiloxane | 250 |
| Isostearyl palmitate | 250 |
| Tocopheryl acetate | 20 |
| Melatonin | 1 |
| 2-phenoxyethanol | 2 |
wherein the percentages of the various ingredients are to be considered as expressed in weight.

14. A pharmaceutical composition for transcutaneous administration which consists essentially of an oil in water emulsion consisting of:
| | |
|---|---|
| Alkylesters of : | |
| Polyethylene glycol (OE = 8) | 100 |
| Cetyl and stearyl alcohol | 15 |
| Glyceryl tristearate | 30 |
| Hydrogenated glycerides of tallow | 5 |
| Alkyl benzoate | 70 |
| Jojoba oil | 15 |
| Melatonin | 1 |
| Dimethylpolysiloxane | 10 |
| Tocopheryl acetate | 10 |
| Magnesium ascorbyl phosphate | 5 |
| Glycerin | 30 |
| 2-phenoxyethanol | 2 |
| Imidazolidinylurea | 3 |
| Deionized water | 1000 |
wherein the percentages of the various ingredients are to be considered as expressed in weight.

15. A pharmaceutical composition for transcutaneous administration which is in the form of an emulsion which consists of:
| | |
|---|---|
| Lauryl dimethylpolysiloxane | |
| Polyethylene glycol copoliolo | 20 |
| Dimethylpolysiloxane | 100 |
| Cyclicdimethylpolysiloxane | 50 |
| Melatonin | 1 |
| Tocopheryl acetate | 2 |
| Alkyl lactate | 47 |
| Polyethylene glycol lactate (OE = 3) | 5 |
| Sodium chloride | 20 |
| Glycerin | 20 |
| Imidazolidinylurea | 2 |
| Deionized water | 1000 |
wherein the percentages of the various ingredients are to be considered as expressed in weight.

16. A pharmaceutical composition for transcutaneous administration which consists of an emulsion consisting of:
| | |
|---|---|
| Cetyldimethylpolysiloxane | |
| Polyethylene glycol copoliolo | |
| cetyldimethylpolysiloxane | |
| polyglyceryl oleate and hexyl laurate | 50 |
| Polyisoprene | 140 |
| Dimethylpolysiloxane | 20 |
| Cyclicdimethylpolysiloxane | 30 |
| Tocopheryl acetate | 1 |
| Melatonin | 1 |
| Sodium chloride | 20 |
| 2-phenoxyethanol | 2 |
| Imidazolidinylurea | 3 |
| Sorbitol | 20 |
| Deionized water | 1000 |
wherein the percentages of the various ingredients are to be considered as expressed in weight.

17. A pharmaceutical composition according to claim 1 which comprises as the active ingredient at least one member selected from the group consisting of melatonin agonists and phospholipids of liposomic, neosomic or non-neosomic structure.

18. A pharmaceutical composition according to claim 1 which additionally comprises at least one solubilizing agent.

19. A pharmaceutical composition according to claim 1 which additionally contains Kappa elastin, said Kappa elastin being the elastin fraction of the protein.

20. The pharmaceutical composition according to claim 1 which additionally comprises at least one member selected from the group consisting of proteins and peptides.

## Patentansprüche

1. Pharmazeutische Zusammensetzung zur transkutanen Verabreichung, die insbesondere bei der Behandlung von Pathologien wirksam ist, die die zirkadianen Rhythmen beeinträchtigen, die als Wirkstoff einen oder mehrere Melatonin-Agonisten gemäß der unten aufgeführten Formel aufweist: 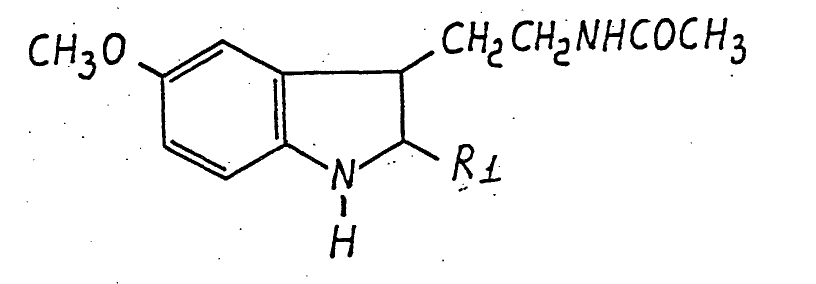 ausgewählt aus:
2-Br-N-Acetyl-5-Methoxytryptamin
2-I-N-Acetyl-5-Methoxytryptamin
2-F-N-Acetyl-5-Methoxytryptamin
2-Cl-N-Acetyl-5-Methoxytryptamin, wobei der Prozentsatz des Wirkstoffes zwischen 0,1 und 5 % V/U liegt.

2. Pharmazeutische Zusammensetzung nach Anspruch 1,
**wobei**
die Zusammensetzung die Form eines Öls aufweist.

3. Pharmazeutische Zusammensetzung nach Anspruch 1,
**wobei**
die Zusammensetzung die Form eines Gels aufweist.

4. Pharmazeutische Zusammensetzung nach Anspruch 1,
**wobei**
die Zusammensetzung die Form einer Paste oder Creme aufweist.

5. Pharmazeutische Zusammensetzung nach Anspruch 1,
**wobei**
die Zusammensetzung auf einen festen Träger aufgebracht wird.

6. Pharmazeutische Zusammensetzung nach Anspruch 5,
**wobei**
der Träger aus Lappen, okkludierter oder nichtokkludierter Gaze besteht.

7. Pharmazeutische Zusammensetzung nach Anspruch 1,
**wobei**
die Zusammensetzung zusätzlich einen Eindringverzögerer aufweist.

8. Pharmazeutische Zusammensetzung nach Anspruch 7,
**wobei**
die Eindringverzögerer Protein-Mikrosphaerulen aus Zellulose sind.

9. Pharmazeutische Zusammensetzung zur transkutanen Verabreichung in Form eines Gels, die besteht aus:
| | |
|---|---|
| Homopolymer aus Acrylsäure | 4 |
| Allylether aus Zucker | 4 |
| Ethanol | 50 |
| Melatonin | 1 |
| Imidazolidinylurea | 3 |
| Glyzerin | 20 |
| Triethanolamin | 30 |
| Gehärtetem Rizinusöl | |
| Polyethylenglykol (OE = 40) | 2 |
| Cyclodimethylpolysiloxan | 1 |
| Benzoat aus Fettalkoholen (aliphatisch) | 1 |
| Isodamascon | 0,3 |
| Entionisiertem Wasser | 1000 |
wobei die Prozentsätze der verschiedenen Wirkstoffe als in Gewicht ausgedrückt betrachtet werden.

10. Pharmazeutische Zusammensetzung zur transkutanen Verabreichung, die insbesondere bei der Behandlung von Pathologien wirksam ist, die die zirkadianen Rhythmen beeinträchtigen, die besteht aus:
| | |
|---|---|
| Homopolymer aus Acrylsäure | 4 |
| Allylether aus Zucker | 4 |
| Ethylalkohol | 320 |
| Melatonin | 10 |
| Imidazolidinylurea | 3 |
| Glyzerin | 20 |
| Triethanolamin | 10 |
| Gehärtetem Rizinusöl | |
| Polyethylenglykol (0E = 40) | 2 |
| Cyclodimethylpolysiloxan | 1 |
| Benzoat aus Fettalkoholen (aliphatisch) | 1 |
| Isodamascon | 0,3 |
| Entionisiertem Wasser | 1000 |
wobei die Prozentsätze der verschiedenen Wirkstoffe als in Gewicht ausgedrückt betrachtet werden.

11. Pharmazeutische Zusammensetzung zur transkutanen Verabreichung, die besteht aus:
| | |
|---|---|
| Homopolymer aus Acrylsäure | 4 |
| Allylether aus Zucker | 4 |
| Ethanol | 303 |
| Melatonin | 30,3 |
| Phospholipiden | 91 |
| Imidazolidinylurea | 3 |
| Glyzerin | 20 |
| Triethanolamin | 10 |
| Gehärtetem Rizinusöl | |
| Polyethylenglykol (0E = 40) | 2 |
| Cyclodimethylpolysiloxan | 1 |
| Benzoat aus Fettalkoholen (aliphatisch) | 1 |
| Isodamascon | 0,3 |
| Entionisiertem Wasser | 1000 |
wobei die Prozentsätze der verschiedenen Wirkstoffe als in Gewicht ausgedrückt betrachtet werden.

12. Pharmazeutische Zusammensetzung zur transkutanen Verabreichung, in Form eines Gels, das besteht aus:
| | |
|---|---|
| Polyglyceryl-Methacrylat | 350 |
| Ethanol | 50 |
| Melatonin | 1 |
| Gehärtetem Rizinusöl | |
| Polyethylenglykol (DE = 40) | 5 |
| 2-Phenoxyethanol | 2 |
| Imidazolidinylurea | 3 |
| Entionisiertem Wasser | 1000 |
wobei die Prozentsätze der verschiedenen Wirkstoffe als in Gewicht ausgedrückt betrachtet werden.

13. Pharmazeutische Zusammensetzung zur transkutanen Verabreichung, in Form eines Öls.
| | |
|---|---|
| Cyclodimethylpolysiloxan | 478 |
| Dimethylpolysiloxan | 250 |
| Isostearyl-Palmitat | 250 |
| Tocopheryl-Acetat | 20 |
| Melatonin | 1 |
| 2-Phenoxyethanol | 2 |
wobei die Prozentsätze der verschiedenen Wirkstoffe als in Gewicht ausgedrückt betrachtet werden.

14. Pharmazeutische Zusammensetzung zur transkutanen Verabreichung, die im wesentlichen aus einer Öl-in-Wasser-Emulsion besteht, die besteht aus:
| | |
|---|---|
| Alkylestern aus: | |
| Polyethylenglykol (OE = 8) | 100 |
| Cetyl- und Stearylalkohol | 15 |
| Glyceroltristearat | 30 |
| Gehärteten Glyceriden aus Talg | 5 |
| Alkylbenzoat | 70 |
| Jojobaöl | 15 |
| Melatonin | 1 |
| Dimethylpolysiloxan | 10 |
| Tocopheryl-Acetat | 10 |
| Magnesium-Ascorbylphosphat | 5 |
| Glyzerin | 30 |
| 2-Phenoxyethanol | 2 |
| Imidazolidinylurea | 3 |
| Entionisiertem Wasser | 1000 |
obei die Prozentsätze der verschiedenen Wirkstoffe als in Gewicht ausgedrückt betrachtet werden.

15. Pharmazeutische Zusammensetzung zur transkutanen Verabreichung, die im wesentlichen die Form einer Emulsion aufweist, die besteht aus:
| | |
|---|---|
| Lauryl-Dimethylpolysiloxan | |
| Polyethylenglycol-Copoliolo | 20 |
| Dimethylpolysiloxan | 100 |
| Zyklischem Dimethylpolysiloxan | 50 |
| Melatonin | 1 |
| Tocopheryl-Acetat | 2 |
| Alkyllactat | 47 |
| Polyethylenglycol-Lactat (0E = 3) | 5 |
| Natriumchlorid | 20 |
| Glyzerin | 20 |
| Imidazolidinylurea | 2 |
| Entionisiertem Wasser | 1000 |
wobei die Prozentsätze der verschiedenen Wirkstoffe als in Gewicht ausgedrückt betrachtet werden.

16. Pharmazeutische Zusammensetzung zur transkutanen Verabreichung, die aus einer Emulsion besteht, die besteht aus:
| | |
|---|---|
| Cetyldimethylpolysiloxan | |
| Polyethylenglycol-Copoliolo- | |
| Cetyldimethylpolysiloxan- | |
| Polyglyceryloleat und Hexyllaurat | 50 |
| Polyisopren | 140 |
| Dimethylpolysiloxan | 20 |
| Zyklischem Dimethylpolysiloxan | 30 |
| Tocopheryl-Acetat | 1 |
| Melatonin | 1 |
| Natriumchlorid | 20 |
| 2-Phenoxyethanol | 2 |
| Imidazolidinylurea | 3 |
| Sorbitol | 20 |
| Entionisiertem Wasser | 1000 |
wobei die Prozentsätze der verschiedenen Wirkstoffe als in Gewicht ausgedrückt betrachtet werden.

17. Pharmazeutische Zusammensetzung nach Anspruch 1, die als Wirkstoff mindestens ein Element aufweist, das aus der Gruppe ausgewählt ist, die aus Melatonin-Agonisten und Phospholipiden liposomischer, neosomischer oder nicht-neosomischer Struktur besteht.

18. Pharmazeutische Zusammensetzung nach Anspruch 1, die zusätzlich mindestens ein Lösungshilfsmittel aufweist.

19. Pharmazeutische Zusammensetzung nach Anspruch 1, die zusätzlich Kappa-Gerüsteiweißstoffe aufweist, wobei Kappa die Gerüsteiweißstoffraktion des Proteins ist.

20. Pharmazeutische Zusammensetzung nach Anspruch 1, die zusätzlich mindestens ein Element aufweist, das aus der aus Proteinen und Peptiden bestehenden Gruppe ausgewählt ist.

## Revendications

1. Composition pharmaceutique pour une administration transcutanée particulièrement efficace dans le traitement de pathologies qui interfèrent avec les rythmes circadiens, qui comprend comme ingrédients actifs au moins un agoniste de mélatonine selon la formule ci-dessous : 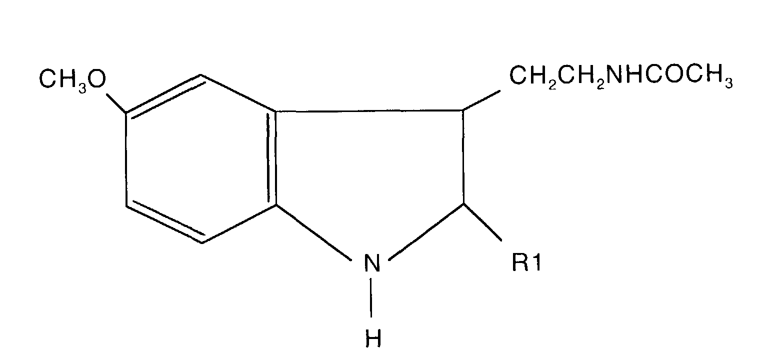 choisi à partir
2-Br-N-acéthyl-5-méthoxytryptamine
2-I-N-acéthyl-5-méthoxytryptamine
2-F-N-acéthyl-5-méthoxytryptamine
2-CI-N-acéthyl-5-méthoxytryptamine, dans lequel le pourcentage dudit ingrédient actif est compris entre 0,1 et 5% v/v.

2. Composition pharmaceutique selon la revendication 1 dans laquelle ladite composition est sous la forme d'une huile.

3. Composition pharmaceutique selon la revendication 1 dans laquelle ladite composition est sous la forme d'un gel.

4. Composition pharmaceutique selon la revendication 1 dans laquelle ladite composition est sous la forme d'une pâte ou d'une crème.

5. Composition pharmaceutique selon la revendication 1 dans laquelle ladite composition est appliquée sur un support solide.

6. Composition pharmaceutique selon la revendication 5 dans lequel ledit support solide est un timbre transdermique (« patch »), une gaze occlusive ou non-occlusive ("occluded or non-occluded gauze").

7. Composition pharmaceutique selon la revendication 1 dans laquelle ladite composition comprend en outre un retardateur de pénétration.

8. Composition pharmaceutique selon la revendication 7 dans laquelle lesdits retardateurs de pénétration sont des micro-sphères de cellulose protéiques.

9. Composition pharmaceutique pour administration transcutanée sous la forme d'un gel qui consiste en :
| | |
|---|---|
| Homopolymère d'acide acrylique | 4 |
| Ether allylique de sucres | 4 |
| Ethanol | 50 |
| Mélatonine | 1 |
| Imidazolidinylurée ("Imidazolidinylurea") | 3 |
| Glycérine | 20 |
| Triéthanolamine | 30 |
| Huile de ricin hydrogénée | |
| Polyéthylène glycol (OE=40) | 2 |
| Cyclodiméthylpolysiloxane | 1 |
| Benzoate d'alcools gras (aliphatique) | 1 |
| Isodamascone ("Isodamascone") | 0.3 |
| Eau déminéralisée | 1000 |
dans lesquels les pourcentages des divers ingrédients sont exprimés en poids.

10. Composition pharmaceutique pour administration transcutanée particulièrement efficace dans le traitement de pathologies qui interfèrent avec les rythmes circadiens qui consistent en :
| | |
|---|---|
| Homopolymère d'acide acrylique | 4 |
| Ether allylique de sucres | 4 |
| Alcool d'éthyle | 320 |
| Mélatonine | 10 |
| Imidazolidinylurée | 3 |
| Glycérine | 20 |
| Triéthanolamine | 10 |
| Huile de ricin hydrogénée | |
| Polyéthylène glycol (OE=40) | 2 |
| Cyclodiméthylpolysiloxane | 1 |
| Benzoate d'alcools gras (aliphatique) | 1 |
| Isodamascone | 0.3 |
| Eau déminéralisée | 1000 |
dans laquelle les pourcentages des différents ingrédients sont exprimés en poids.

11. Composition pharmaceutique pour administration transcutanée qui consiste en :
| | |
|---|---|
| Homopolymère d'acide acrylique | 4 |
| Ether allylique de sucres | 4 |
| Ethanol | 303 |
| Mélatonine | 30.3 |
| Phospholipides | 91 |
| Imidazolidinylurée | 3 |
| Glycérine | 20 |
| Triéthanolamine | 10 |
| Huile de ricin hydrogénée | |
| Polyéthylène glycol (OE=40) | 2 |
| Cyclodiméthylpolysiloxane | 1 |
| Benzoate d'alcools gras (aliphatique) | 1 |
| Isodamascone | 0.3 |
| Eau déminéralisée | 1000 |
dans laquelle les pourcentages des divers ingrédients sont exprimés en poids.

12. Composition pharmaceutique pour administration transcutanée qui est sous la forme d'un gel consistant en :
| | |
|---|---|
| Méthacrylate de polyglycéryle | 350 |
| Ethanol | 50 |
| Mélatonine | 1 |
| Huile de ricin hydrogénée | |
| Polyéthylène glycol (DE=40) | 5 |
| 2-phénoxyéthanol | 2 |
| Imidazolidinylurée | 3 |
| Eau déminéralisée | 1000 |
dans laquelle les pourcentages des divers ingrédients sont exprimés en poids.

13. Composition pharmaceutique pour administration transcutanée qui est sous la forme d'une huile.
| | |
|---|---|
| Cyclodiméthylpolysiloxane | 478 |
| Diméthylpolysiloxane | 250 |
| Palmitate d'isostéaryle | 250 |
| Acétate de Tocophéryle ("Tocopheryl acetate") | 20 |
| Mélatonine | 1 |
| 2-phénoxyéthanol | 2 |
dans laquelle les pourcentages des différents ingrédients sont exprimés en poids.

14. Composition pharmaceutique pour administration transcutanée qui consiste essentiellement en une huile dans une émulsion d'eau consistant en :
| | |
|---|---|
| Alkylesters de : | |
| Polyéthylène glycol (OE=8) | 100 |
| Alcool hexadécyclique ("cetyl") et stéaryle | 15 |
| Tristéarate de glycéryle | 30 |
| Glycérides hydrogénées de suif (« tallow ») | 5 |
| Benzoate d'alkyle | 70 |
| Huile de jojoba | 15 |
| Mélatonine | 1 |
| Diméthylpolysiloxane | 10 |
| Acétate de tocophéryle | 10 |
| Phosphate d'ascorbyle de magnésium | 5 |
| Glycérine | 30 |
| 2-phénoxyéthanol | 2 |
| Imidazolidinylurée | 3 |
| Eau déminéralisée | 1000 |
dans laquelle les pourcentages des divers ingrédients sont exprimés en poids.

15. Composition pharmaceutique pour administration transcutanée qui sous la forme d'une émulsion qui consiste en :
| | |
|---|---|
| Diméthylpolysiloxane laurique | |
| Polyéthylène glycol copoliolo ("Polyethylene glycol copoliolo") | 20 |
| Diméthylpolysiloxane | 100 |
| Diméthylpolysiloxane cyclique | 50 |
| Mélatonine | 1 |
| Acétate de tocophéryle | 2 |
| Lactate d'alkyl | 47 |
| Lactate de polyéthylène glycol (OE=3) | 5 |
| Chlorure de sodium | 20 |
| Glycérine | 20 |
| Imidazolidinylurée | 2 |
| Eau déminéralisée | 1000 |
dans laquelle les pourcentages des divers ingrédients sont exprimés en poids.

16. Composition pharmaceutique pour administration transcutanée qui consiste en une émulsion consistant en :
| | |
|---|---|
| Diméthylpolyciloxane hexadécyclique ("Cetyldimethylpolysiloxane") | |
| Polyéthylène glycol copoliolo | |
| Diméthylpolysiloxane hexadécyclique | |
| Oléate de polyglycéryl et hexylique laurique | 50 |
| Polyisoprène | 140 |
| Diméthylpolysiloxane | 20 |
| Diméthylpolyciloxane cyclique | 30 |
| Acétate de tocophéryle | 1 |
| Mélatonine | 1 |
| Chlorure de sodium | 20 |
| 2-phénoxyéthanol | 2 |
| Imidazolidinylurée | 3 |
| Sorbitol | 20 |
| Eau déminéralisée | 1000 |
dans laquelle les pourcentages des divers ingrédients sont exprimés en poids.

17. Composition pharmaceutique selon la revendication 1 qui comprend comme ingrédient actif au moins un membre choisi dans le groupe formé par les agonistes de mélatonine et les phospholipides de structure liposomique, néosomique, ou non-néosomique.

18. Composition pharmaceutique selon la revendication 1 qui comprend en outre au moins un agent de solubilisation.

19. Composition pharmaceutique selon la revendication 1 qui contient en outre l'élastine Kappa ("Kappa elastin"), ladite élastine Kappa étant la fraction d'élastine de la protéine.

20. Composition pharmaceutique selon la revendication 1 qui comprend en outre au moins un membre choisi à partir du groupe formé par les protéines et les peptides.
